# EUROPEAN PATENT SPECIFICATION

(11) **EP 1 650 224 B1**
(45) Date of publication and mention of the grant of the patent: **06.03.2013**
(21) Application number: 05256462.2
(22) Date of filing: 18.10.2005
(51) Int. Cl.: C07K 14/475, A61L 24/10, A61K 47/42, A61K 38/36

(54) **Composition and method for repairing nerve damage and enhancing functional recovery of nerve**
Zusammensetzung und Methode für die Reparatur von Nervenschädigung und das Erhöhen der Funktionswiederaufnahme des Nervs
Composition et méthode pour réparer des dommages de nerf et augmenter le rétablissement fonctionnel du nerf

(30) Priority: 18.10.2004 US 967907
(43) Date of publication of application: 26.04.2006
(73) Proprietor: Taipei Veterans General Hospital, Taipei 112 (TW)
(72) Inventor: Cheng, Henrich, Pei-Tou Dist, Taipei (TW); Huang, Shiang-Suo, Taipei (TW); Tsai, Shen-Kou, Pei-Tou Dist., Taipei (TW)
(74) Representative: Sexton, Jane Helen

(56) References cited:
- WO-A-03/077917
- US-A1- 2004 138 155
- US-A1- 2004 267 289
- RUMMLER L S ET AL: "Peripheral nerve repair: A review" CURRENT OPINION IN ORTHOPAEDICS 2004 UNITED STATES, vol. 15, no. 4, 2004, pages 215-219, XP009060269 ISSN: 1041-9918
- JUBRAN MARIE ET AL: "Repair of peripheral nerve transections with fibrin sealant containing neurotrophic factors." EXPERIMENTAL NEUROLOGY, vol. 181, no. 2, June 2003 (2003-06), pages 204-212, XP002364196 ISSN: 0014-4886
- CHENG HENRICH ET AL: "Characterization of a fibrin glue-GDNF slow-release preparation" CELL TRANSPLANTATION, vol. 7, no. 1, January 1998 (1998-01), pages 53-61, XP002364197 ISSN: 0963-6897
- IWAKAWA M ET AL: "Intraspinal implants of fibrin glue containing glial cell line-derived neurotrophic factor promote dorsal root regeneration into spinal cord." NEUROREHABILITATION AND NEURAL REPAIR. 2001, vol. 15, no. 3, 2001, pages 173-182, XP009060272 ISSN: 1545-9683
- CHENG HENRICH ET AL: "Spinal cord repair with acidic fibroblast growth factor as a treatment for a patient with chronic paraplegia" SPINE, vol. 29, no. 14, 15 July 2004 (2004-07-15), pages E284-E288, XP009060268 ISSN: 0362-2436
- CHENG H ET AL: "The neuroprotective effect of glial cell line-derived neurotrophic factor in fibrin glue against chronic focal cerebral ischemia in conscious rats" BRAIN RESEARCH, AMSTERDAM, NL, vol. 1033, no. 1, 1 February 2005 (2005-02-01), pages 28-33, XP004745030 ISSN: 0006-8993
- SPOTNITZ WILLIAM D ET AL: "Fibrin sealant tissue adhesive--review and update." JOURNAL OF LONG-TERM EFFECTS OF MEDICAL IMPLANTS. 2005, vol. 15, no. 3, 2005, pages 245-270, XP009060270 ISSN: 1050-6934

## Description

### BACKGROUND OF THE INVENTION

### 1. Field of the Invention

The invention relates to a composition and the composition for use in a method for repairing a nerve damage and enhancing functional recovery of a damaged nerve.

### 2. Description of the Related Art

Nerve damage is usually caused by trauma or ischemia, and is difficult to repair. A vertebrate having nerve damage may suffer from motor deficits, paralysis, or even death.

Many strategies have been developed to repair nerve damage, one of which is the use of neurotrophic factors. Glial cell line-derived neurotrophic factor (GDNF), a member of the transforming growth factor-β (TGF-β) superfamily, is considered the most potent neurotrophic factor that promotes survival and neurite outgrowth of dopaminergic neurons and motoneurons as well as peripheral sensory and sympathetic neurons. See Science, 260:1130-1132 (1993); Science, 266:1062-1064 (1994); Nature, 373:289-290 (1995); Nature, 373: 335-339 (1995); Nature, 373: 341-344 (1995); and Nature, 373: 344-346 (1995).

Middle cerebral artery (MCA) occlusion causes not only a wide range of infarction but impairment in motor performance. The neuronal damage in the territory of MCA, e.g., caudate putamen and striatum, will cause motor deficits. See Stroke, 28:2060-2066 (1997). GDNF gene expression was shown to be transiently induced in the ipsilateral cortex and caudate at the early stage of focal brain ischemia. See Brain Res., 776:230-234. Furthermore, the effect of GDNF on the changes of infarct size, brain edema, DNA fragmentation, and immunoreactivities for caspases after permanent MCA occlusion in rats has been investigated. See Stroke, 29:1417-1422 (1998). It is also reported that the topical application of GDNF on ischemic brain surface significantly reduced the size of infarction and the brain edema of reperfused rat brain after acute FCI injury. See Neurosci Lett., 231:37-40 (1997).

However, the topical application of GDNF is shown to reduce infarction size in a time-dependent manner, and the therapeutic time windows was shorter than other chemical compounds, such as MK-801, an NMDA (N-methyl-D-aspartate) receptor antagonist, and the free radical scavenger, alpha-phenyl-tert-butyl-nitrone (PBN). See Brain Res., 903:253-256 (2001).

The direct application of GDNF is not effective for some nerve damages, especially chronic nerve damages. For example, GDNF has not been effective against chronic FCI injury. See Hum Gene Ther.13:1047-1059 (2002).

GDNF is reported to be able to be slowly released in physiologically relevant amounts over a long-acting period of time. See Exp Brain Res., 104:199-206 (1995); and Cell Transplant., 7:53-61 (1998). Nevertheless, slow release of GDNF cannot successfully treat chronic nerve damages, or enhance the functional recovery of nerves.

There remains a need for a means for effectively repairing nerve damages and in furtherance, enhancing the functional recovery of damaged nerves.

### SUMMARY OF THE INVENTION

The purpose of the invention is to provide a method for effectively repairing nerve damages and in furtherance, enhancing the functional recovery of damaged nerves.

It is surprisingly found that the purpose of the invention can be fulfilled with a fibrin glue composition which comprises glial cell line-derived neurotrophic factor (GDNF), fibrinogen, aprotinin and divalent calcium ions.

Accordingly, in the first aspect, the invention provides glial cell line-derived neurotrophic factor (GDNF) for use in a fibrin glue composition comprising GDNF, fibrinogen, aprotinin and divalent calcium ions, for use in a method of repairing nerve damage of a central nervous system nerve caused by an ischemic brain disease and/or enhancing the functional recovery of the damaged nerve.

In a further aspect, the invention provides use of glial cell line-derived neurotrophic factor (GDNF) in the manufacture of a fibrin glue composition comprising GDNF, fibrinogen, aprotinin and divalent calcium ions for repairing nerve damage of a central nervous system nerve caused by an ischemic brain disease and/or enhancing functional recovery of the damaged nerve.

In an even further aspect, the invention provides a kit consisting essentially of GDNF, fibrinogen, aprotinin and a source of divalent calcium ions, for use in the preparation of a fibrin glue composition comprising GDNF, fibrinogen, aprotinin and a source of divalent calcium ions, for use in repairing nerve damage of a central nervous system nerve caused by an ischemic brain disease or for enhancing the functional recovery of the damaged verve.

A method is described for enhancing the functional recovery of nerves, which comprises topically applying to a damaged nerve the fibrin glue composition of the invention.

### BRIEF DESCRIPTION OF THE DRAWINGS

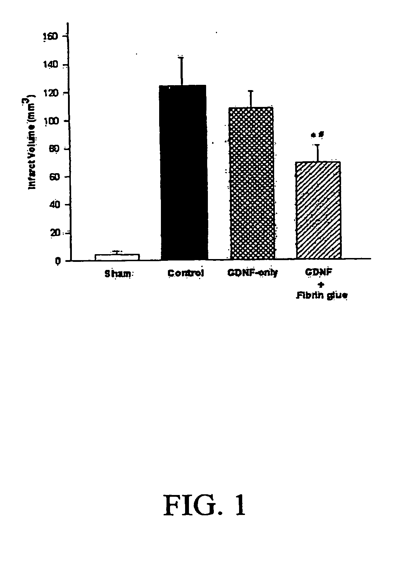

FIG. 1. Infarct volumes assessed after one hour MCA occlusion and four weeks reperfusion by vital TTC staining. The infarct volume of the GDNF-fibrin glue group was significantly reduced as compared with the control group and the GDNF-only group. Each experimental group consisted of six rats. Results are expressed as mean ± SEM. *, P<0.05 when compared with the control group. ^{#}, P<0.05 when compared with the GDNF-only group.

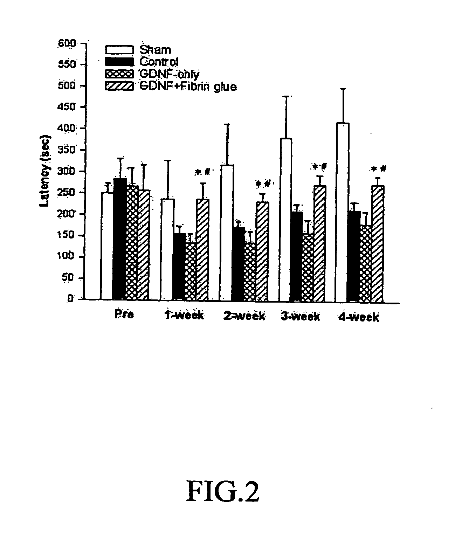

FIG. 2. Effect of GDNF on rotarod test. Duration of time (in sec) spent on the rotarod test. Sham-operated animals without ischemia stayed significantly longer on the rotarod test than other groups. In the GDNF-fibrin glue group, the duration of time when rats stayed on the rotarod were significant increase as compared with the control group and the GDNF-only group at the end of 1st, 2nd, 3rd and 4th week after focal cerebral ischemia. Each experimental group consisted of six rats. Results are expressed as mean ± SEM. *, P<0.05 when compared with the control group. ^{#}, P<0.05 when compared with the GDNF-only group.

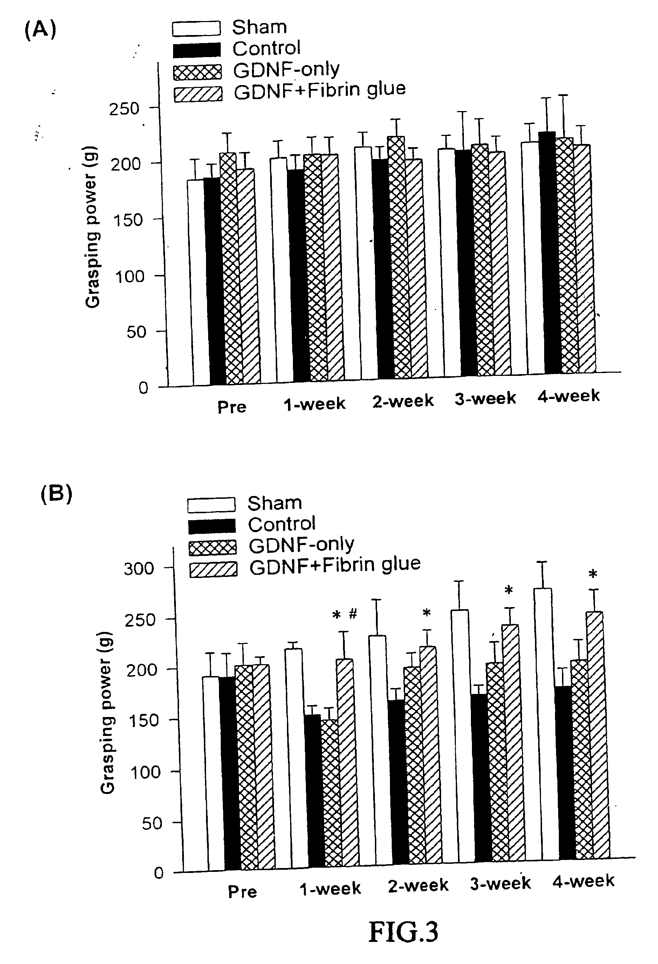

FIG. 3. Effect of GDNF on grasping power of (A) right forepaw and (B) left forepaw of rats at the end of the 1st, 2nd, 3rd, and 4th week after FCI injury. Each experimental group consisted of six rats. Results are expressed as mean ± SEM. *, P<0.05 when compared with the control group. ^{#}, P<0.05 when compared with the GDNF-only group.

### DETAILED DESCRIPTION OF THE INVENTION

The invention relates to a fibrin glue composition for repairing a nerve damage, and/or enhancing the functional recovery of a damaged nerve which comprises an effective amount of nerve growth factor, fibrinogen, aprotinin and divalent calcium ions; wherein the nerve growth factor is a glial cell line-derived neurotrophic factor.

The term "fibrin glue" as used herein refers to a biocompatible and biodegradable product formed by fibrinogen and other reagents.

The term "effective amount" as used herein refers to an amount of the active ingredients of the fibrin glue composition of the invention, which, when administered to a subject suffering from nerve damages, attains a desired effect, i.e., repairs nerve damages in the subject, and/or enhances the functional recovery of damaged nerves. The effective amount can be readily determined by persons of ordinary skills of the art.

According to the invention, the concentration of nerve growth factor in the fibrin glue composition of the invention may preferably be in the range of about 1 to 1000 µg/ml of the composition, more preferably 50 µg/ml.

According to the invention, the concentration of fibrinogen in the fibrin glue composition of the invention may preferably be in the range of about 10 to 1000 mg/ml, more preferably about 100 mg/ml.

According to the invention, the concentration of aprotinin in the fibrin glue composition of the invention may preferably be in the range of about 10 to 500 KIU/ml of the composition, more preferably 200 KIU/ml.

According to the invention, the divalent calcium ions can derive from any calcium ion sources, such as those provided by calcium chloride or calcium carbonate. The concentration of the calcium chloride in the fibrin glue composition of the invention may preferably be in the range about 1 to 100 mM, more preferably 8 mM.

The fibrin glue of the invention can be conveniently prepared by mixing the ingredients. For instance, nerve growth factor and fibrinogen is mixed with aprotinin solution and then the resultant mixture is further mixed with a calcium source. The resultant composition can be stored before use. Alternatively, the composition can also be freshly prepared right before use. Preferably, the fibrin glue is freshly prepared before application.

The fibrin glue composition of the invention is suitable for use in repairing all kinds of nerve damages and enhancing the functional recovery of the damaged nerves in all nerve systems, including the central nervous system, peripheral nervous system, sympathetic nervous system, and parasympathetic nervous system. According to the claimed invention, the nerve is a central nervous system nerve. In an embodiment of the invention, the nerve damage is caused by focal cerebral ischemia (FCI). In another embodiment, the nerve damage is caused by chronic focal cerebral ischemia.

As used herein, the term "repairing a nerve damage(s)" refers to an improvement in the pathological conditions of the subject suffering from nerve damages, and the term "functional recovery of nerves" refers to restoration of the physical function of damaged nerves. For example, for nerve damages caused by FCI, the reduction in total infarction volume and motor deficits are signs of the repair of the damaged nerves and restoration of the function thereof. In an animal model according to the invention, the reduction in motor deficits comprises improvement in balance, coordination and grasping strength.

The fibrin glue composition of the invention can be topically applied to damaged nerves. The topical application may be practiced during a surgery.

The method using the composition of the invention exerts a long-term effect needed for repairing chronic nerve damages and enhancing the functional recovery of the damaged nerves. In addition, topical application of the composition of the invention to damaged nerves for only once can sufficiently achieve the desired effect.

Not wishing to be bound by theory, it is believed that the effect of the fibrin glue of the invention in repairing nerve damages and/or enhancing functional recovery of nerves does not result from the slow release of nerve growth factor, but from other unknown mechanisms.

In view of the effects on damaged nerves, the compositions and/or uses of the invention can effectively treat nerve damages-related diseases, including, but not limited to, ischemic brain disease. Preferably, the ischemic brain disease comprises stroke, thrombosis and embolization of MCA.

As shown in the examples, *infra*, while topical application of the fibrin glue composition of the invention to ischemic brain significantly decreases the infarction volume in MCA occluded rats and also significantly improves the motor performance at the end of 4th week after chronic FCI injure, the control group did not produce neuroprotective effect in rats after chronic focal cerebral ischemia. The data suggest that the fibrin glue composition of the invention is effective in treating the cerebral ischemia from MCA occlusion and enhancing recovery of motor function.

The following Examples are given for the purpose of illustration only and are not intended to limit the scope of the present invention.

### Example 1: Method for Repairing Nerve Damage and Enhancing Functional Recovery

*Animals:* The example conforms to the Guide for the Care and Use of Laboratory Animals, published by the US National Institutes of Health (NIH publication NO. 85-23, revised 1996). Male Long-Evans rats (National Lab. Animal Breeding and Research Center) weighing 300-350g were used in this study. These animals were housed in a room with controlled temperature (24±1°C) and humidity (55±5%) under a 12:12 h light-dark cycle. They were allowed free access to food and water.

*Surgical procedure:* The technique was a modification of the method of Huang *et al* (Huang SS, Tsai SK, Chih CL, Chiang LY, Hsieh HM, Teng CM, Tsai MC. Neuroprotective effect of hexasulfobutylated C60 on rats subjected to focal cerebral ischemia. Free Radic Biol Med. 2001;30:643-649). In brief, each male Long-Evans rat was anesthetized by inhalation of a nitrous oxide/oxygen/halothane (69%:30%:1%) mixture during surgical preparation. Body temperature was maintained during surgery at 37±0.5°C with a heating pad servo-controlled by a rectal probe. The ventral tail artery was cannulated for continuous monitoring of heart rate and mean arterial blood pressure (MABP) by Statham™ P23 XL transducer and displayed on a Gould RS-3400 physiological Recorder (Gould®, Cleveland, OH, USA) and the pH, Po₂ and P_{CO₂} in the blood were tested using blood sampling with Blood Gas Analyzer (GEM-5300 I.L. CO®, USA). Measurements were performed before, during and right after unilateral MCA occlusion.

Focal ischemic infarcts were produced in the right lateral cerebral cortex in the territory of the MCA. Both common carotid arteries were exposed by midline anterior cervical incision. The animal was placed in a lateral position, and a skin incision was made at the midpoint between the right lateral canthus and the anterior pinna. The temporal muscle was retracted, and a small (3-mm diameter) craniectomy was made at the junction of the zygoma and squamosal bone using a drill (Dremel™ Multipro+5395, Dremel com®. USA) cooled with saline solution. Using a dissecting microscope (OPMI-1, ZISS®, Germany), the dura was opened with fine forceps, and the right MCA was ligated with 10-0 monofilament nylon ties. Both common carotid arteries were then occluded by microaneurysm clips for 1 hr. After removing the clips, return of flow was visualized in the arteries.

*Experimental groups:* After FCI injury, rats were assigned in randomized sequence to one of four treatment groups (n=6 each): (a) topically applied GDNF (RBI, Natick®, MA, USA)-fibrin glue group:
GDNF (1 µg) was mixed with fibrinogen plus aprotinin solution. (b) topically applied GDNF-only group: GDNF (1 µg) was dissolved in HBSS solution. (c) Control group: the fibrin glue was made by adding HBSS solution. (d) Sham group: animals underwent the same described surgical procedures except MCA ligation.

Fibrin glue (Beriplast P™, Germany) used as adhesive agent in CNS tissue and was customarily prepared before use by mixing the fibrinogen (100 mg/ml) with aprotinin solution (200 KIU/ml). This solution was further mixed with calcium chloride (8 mM) in the surgical area to form a glue cast. The final volume for locally application to infarcted brain tissue was 20 µl.

*Osmotic applied GDNF group:* 1 µg GDNF (RBI, Natick®, MA, USA) was dissolved in HBSS solution. (n=6). Rats were implanted with guide cannulae for the drug infusion. Rats were anesthetized and a 21-gauge stainless steel cannula was implanted in the right lateral ventricle of the rat brain. The cannula was held in place with rapid-setting dental acrylic (Lang Dental®, Wheeling, IL, USA) anchored to the skull by an aluminum protective cap and steel screws. The minipump was implanted s.c. as described [referece osmotic] with minor modification. Briefly, a small cut was made behind the ears of the rats and the subcutaneous space was expanded with a hemostatic forceps. HBSS solution or GDNF (1 µg in HBSS solution) was filtered through a 0.2-mm sterile syringe filter (Sterile Acrodisc) and was then used to fill an osmotic minipump (Alzet™ 2004, Alza®, Palo Alto, CA, USA). The minipump was implanted and connected directly to the cannula via 6-cm long PE-60 polyethylene tubing. The infusion rate was 0.25 µl per h for 28 days. The incision on the back was closed with cyanoacrylate glue, and dental acrylic was layered on top of the polyethylene tube.

*Infarct volume analysis:* After focal cerebral ischemia for 1 hr and reperfusion for 4 weeks, then the rats were anesthetized and killed by rapid decapitation. Brains were removed, inspected visually for the anatomy of the MCA and for signs of hemorrhage or infection, immersed in cold saline solution for 10 minutes, and sectioned into standard coronal slices (each 2-mm thick) using a brain matrix slicer (JACOBOWITZ™ Systems, Zivic-Miller Laboratories INC®, Allison park, USA). Slices were placed in the vital dye 2,3,5-triphenyltetrazolium chloride (TTC, 2%; Sigma, USA) at 37°C in the dark for 30 minutes, followed by 10% formalin at room temperature overnight. The outline of right and left cerebral hemispheres as well as that of infarct tissue, clearly visualizable by TTC staining (Chen ST, Hsu CY, Hogan EL, Maricq H, Balentine JD. A model of focal ischemic stroke in the rat: reproducible extensive cortical infarction. Stroke. 1986;17:738-743), was outlined on the posterior surface of each slice using an image analyzer (color image scanner, EPSON™ GT-9000), connected to an image analysis system (AIS software, Imaging research INC®, Canada) running on a personal computer, AMD™ K6-2 3D 400. The area of infarction was measured by subtracting the area of the non-lesioned ipsilateral hemisphere from that of the contralateral side. Infarct volume was calculated as the sum of infarct area per slice multiplied by slice thickness. Both the surgeon and image analyzer operator were blind to the treatment given to each animal.

Reproducible brain infarcts were obtained from territory of right MCA occlusion in the GDNF-fibrin glue group, the GDNF-only group and the control group. The infarct volume at the end of 4th week after FCI injury in the GDNF-fibrin glue group was significantly reduced (69.1±12.4 mm³, P<0.05) but not in the GDNF-only group (108.0±12.1 mm³), as compared with the control group (124.0±20.0 mm³) (FIG.1).

The results indicated that topically applied GDNF mixing in fibrin glue at infarct brain tissue after FCI injury significantly reduced the total infarcted volume by 44.3% and 36%, respectively compared to that of control group and GDNF-only group.

*Behavioral tests*: Behavioral measurements were performed by the rotarod test and the grasping power test at the end of 1st, 2nd, 3rd, and the 4th week after focal FCI injury.

### Rotarod test:

The accelerating rotarod was used to assess motor deficit following ischemic injury in rats (Hamm RJ, Pike BR, O'Dell DM, Lyeth BG, Jenkins LW. The rotarod test: an evaluation of its effectiveness in assessing motor deficits following traumatic brain injury. J Neurotrauma. 1994;11:187-196). The rats were placed on rungs of the accelerating rotarod and the time the animals remained on the rotarod was measured. The speed was increased slowly from 4 rev/min to 40 rev/min over the course of 5 minutes. The time in seconds at which each animal fell off the rungs was recorded. Each animal received three consecutive trials.

The results of rotarod test at the four groups are shown in FIG. 2. The duration of animals staying on the rotarod was not significantly different among the four groups before surgical preparation. After FCI injury, the mean latencies for rats to stay on the rotarod were 55.0%, 50.3% and 92.2% (p<0.05 vs. control group and GDNF-only group) of baseline respectively in the control, GDNF-only and GDNF-fibrin glue groups at the end of 1st week after CFI injury but 75.3%, 67.3% and 106.6% (p<0.05 vs. control group and GDNF-only group) of baseline at the end of 4th week after FCI injury. The results showed that after FCI injury, the amount of time for rats staying on the rotarod was significantly shorter in the control and the GDNF-only groups than that of GDNF-fibrin glue animals. This result indicated that cerebral injury area covered with fibrin glue containing GDNF could improve the balance and coordination of rats after FCI injury.

### Grasping power test:

The grasping power test was a modification of the method of Bertelli *et al* (Bertelli JA, Mira JC. The grasping test: a simple behavioral method for objective quantitative assessment of peripheral nerve regeneration in the rat. J Neurosci Methods. 1995;59:151-155). For the assessment of grasping strength, a bar of wires was connected to an ordinary electronic balance. Both forepaws were tested, the untested forepaw was temporarily prevented by wrapping it with adhesive tape, and the tested forepaw was kept free. Rats were allowed to grasp the bar while being lifted by the tail with increasing firmness until they loosened their grip and the grasping power was scored.

The effects on grasping power test of the four groups are shown in FIG. 3. Among the four groups, there is no significant difference in the means of grasping power of left forepaw of rats before the right MCA occlusion and right forepaw of rats before and at the end of the 1st, 2nd, 3rd, and 4th week after right MCA occlusion.

The mean value of grasping powers were 78.7%, 71.7% and 101.2% (p<0.05 vs. control group and GDNF-only group) of baseline respectively in the control, GDNF-only and GDNF-fibrin glue groups at the end of 1st week after FCI injury but 89.6%, 97.6% and 120.7% (p<0.05 vs. control group) of baseline at the end of 4th week after FCI injury.

This result showed that topical application of GDNF-fibrin glue is associated with improvement of grasping strength of rats after FCI injury.

*Statistics:* Data are expressed as mean ± standard error of mean (S.E.M.). Statistical analysis of differences in volume of infarcts and the behavioural deficit score of rats were carried by unpaired, two-tailed t tests, and by two-way analysis of variance (ANOVA) for combined data to evaluate the differences between the control and the treatment groups. A value of P < 0.05 was considered to be statistically significant.

While embodiments of the present invention have been illustrated and described, various modifications and improvements can be made by persons skilled in the art. It is intended that the present invention is not limited to the particular forms as illustrated, and that all the modifications not departing from the scope of the present invention are within the scope as defined in the appended claims.

## Claims

1. Glial cell line-derived neurotrophic factor (GDNF) for use in a fibrin glue composition comprising GDNF, fibrinogen, aprotinin and divalent calcium ions, for use in a method of repairing nerve damage of a central nervous system nerve caused by an ischemic brain disease and/or enhancing the functional recovery of the damaged nerve.

2. GDNF according to Claim 1, wherein the nerve damage is caused by focal cerebral ischemia.

3. GDNF according to Claim 2, wherein the focal cerebral ischemia is chronic focal cerebral ischemia.

4. GDNF according to Claim 1 wherein the ischemic brain disease comprises stroke, thrombosis or embolization of middle cerebral artery.

5. GDNF according to any one of the preceding claims, wherein the divalent calcium ions in the fibrin glue composition derive from calcium chloride or calcium carbonate.

6. GDNF according to any one of the preceding claims wherein the fibrin glue composition is prepared an hour or less before use.

7. Use of glial cell line-derived neurotrophic factor (GDNF) in the manufacture of a fibrin glue composition comprising GDNF, fibrinogen, aprotinin and divalent calcium ions for repairing nerve damage of a central nervous system nerve caused by an ischemic brain disease and/or enhancing functional recovery of the damaged nerve.

8. The use according to claim 7, wherein the nerve damage is caused by focal cerebral ischemia.

9. The use according to claim 8, wherein the focal cerebral ischemia is chronic focal cerebral ischemia.

10. The use according to claim 7, wherein the ischemic brain disease comprises stroke, thrombosis or embolization of middle cerebral artery.

11. The use according to any one of claims 7 to 10, wherein the fibrin glue composition of the invention is freshly prepared an hour or less before use.

12. A kit consisting essentially of GDNF, fibrinogen, aprotinin and a source of divalent calcium ions, for use in the preparation of a fibrin glue composition comprising GDNF, fibrinogen, aprotinin and a source of divalent calcium ions, for use in repairing nerve damage of a central nervous system nerve caused by an ischemic brain disease or for enhancing the functional recovery of the damaged verve.

13. A kit as claimed in claim 12 for use in the preparation of an effective amount of a fibrin glue composition immediately before topical application to a damaged nerve.

14. A kit as claimed in claim 12 or 13 comprising separately GDNF, fibrinogen, aprotinin and a source of divalent calcium ions.

## Patentansprüche

1. Gliazelllinien-abgeleiteter neurotropher Faktor (GDNF) zur Verwendung in einer Fibrinkleber-Zusammensetzung, die GDNF, Fibrinogen, Aprotinin und divalente Calciumionen umfasst, zur Verwendung in einem Verfahren zur Reparatur von Nervenschädigung eines Nervs des zentralen Nervensystems, verursacht durch eine ischämische Gehirnerkrankung, und/oder zur Erhöhung der funktionalen Erholung des geschädigten Nervs.

2. GDNF gemäß Anspruch 1, wobei die Nervenschädigung durch fokale zerebrale Ischämie verursacht ist.

3. GDNF gemäß Anspruch 2, wobei die fokale zerebrale Ischämie chronische fokale zerebrale Ischämie ist.

4. GDNF gemäß Anspruch 1, wobei die ischämische Gehirnerkrankung Schlaganfall, Thrombose oder Embolie der mittleren Zerebralarterie umfasst.

5. GDNF gemäß einem der vorhergehenden Ansprüche, wobei die divalenten Calciumionen in der Fibrinkleber-Zusammensetzung von Calciumchlorid oder Calciumcarbonat abgeleitet sind.

6. GDNF gemäß einem der vorhergehenden Ansprüche, wobei die Fibrinkleber-Zusammensetzung eine Stunde oder weniger vor Verwendung hergestellt wird.

7. Verwendung eines Gliazelllinien-abgeleiteten neurotrophen Faktors (GDNF) zur Herstellung einer Fibrinkleber-Zusammensetzung, die GDNF, Fibrinogen, Aprotinin und divalente Calciumionen umfasst, zur Reparatur von Nervenschädigung eines Nervs des zentralen Nervensystems, verursacht durch eine ischämische Gehirnerkrankung, und/oder zur Erhöhung der funktionalen Erholung des geschädigten Nervs.

8. Verwendung gemäß Anspruch 7, wobei die Nervenschädigung durch fokale zerebrale Ischämie verursacht ist.

9. Verwendung gemäß Anspruch 8, wobei die fokale zerebrale Ischämie chronische fokale zerebrale Ischämie ist.

10. Verwendung gemäß Anspruch 7, wobei die ischämische Gehirnerkrankung Schlaganfall, Thrombose oder Embolie der mittleren Zerebralarterie umfasst.

11. Verwendung gemäß einem der Ansprüche 7 bis 10, wobei die Fibrinkleber-Zusammensetzung der Erfindung eine Stunde oder weniger vor Verwendung frisch hergestellt wird.

12. Kit, der im Wesentlichen aus GDNF, Fibrinogen, Aprotinin und einer Quelle von divalenten Calciumionen besteht, zur Verwendung zur Herstellung einer Fibrinkleber-Zusammensetzung, die GDNF, Fibrinogen, Aprotinin und eine Quelle von divalenten Calciumionen umfasst, zur Verwendung zur Reparatur von Nervenschädigung eines Nervs des zentralen Nervensystems, verursacht durch eine ischämische Gehirnerkrankung, oder zur Erhöhung der funktionalen Erholung des geschädigten Nervs.

13. Kit gemäß Anspruch 12 zur Verwendung zur Herstellung einer wirksamen Menge einer Fibrinkleber-Zusammensetzung unmittelbar vor topischer Anwendung bei einem geschädigten Nerv.

14. Kit gemäß Anspruch 12 oder 13, der GDNF, Fibrinogen, Aprotinin und eine Quelle von divalenten Calciumionen separat umfasst.

## Revendications

1. Facteur GDNF (facteur neurotrophique dérivé d'une lignée de cellules gliales) pour utilisation dans une composition de colle de fibrine comprenant du facteur GDNF, du fibrinogène, de l'aprotinine et des ions de calcium divalents, pour utilisation dans un procédé ayant pour but de réparer des dommages causés à un nerf du système nerveux central par une maladie ischémique cérébrale et/ou de favoriser la récupération fonctionnelle du nerf endommagé.

2. Facteur GDNF conforme à la revendication 1, pour lequel le nerf a été endommagé à cause d'une ischémie cérébrale focale.

3. Facteur GDNF conforme à la revendication 2, pour lequel l'ischémie cérébrale focale est une ischémie cérébrale focale permanente.

4. Facteur GDNF conforme à la revendication 1, pour lequel la maladie ischémique cérébrale comprend une attaque, une thrombose ou une embolisation de l'artère cérébrale moyenne.

5. Facteur GDNF conforme à l'une des revendications précédentes, pour lequel les ions de calcium divalents présents dans la composition de colle de fibrine proviennent de chlorure de calcium ou de carbonate de calcium.

6. Facteur GDNF conforme à l'une des revendications précédentes, pour lequel la composition de colle de fibrine est préparée une heure ou moins avant d'être utilisée.

7. Utilisation du facteur GDNF (facteur neurotrophique dérivé d'une lignée de cellules gliales) dans la fabrication d'une composition de colle de fibrine comprenant du facteur GDNF, du fibrinogène, de l'aprotinine et des ions de calcium divalents, destinée à réparer des dommages causés à un nerf du système nerveux central par une maladie ischémique cérébrale et/ou à favoriser la récupération fonctionnelle du nerf endommagé.

8. Utilisation conforme à la revendication 7, pour laquelle le nerf a été endommagé à cause d'une ischémie cérébrale focale.

9. Utilisation conforme à la revendication 8, pour laquelle l'ischémie cérébrale focale est une ischémie cérébrale focale permanente.

10. Utilisation conforme à la revendication 7, pour laquelle la maladie ischémique cérébrale comprend une attaque, une thrombose ou une embolisation de l'artère cérébrale moyenne.

11. Utilisation conforme à l'une des revendications 7 à 10, dans laquelle la composition de colle de fibrine est préparée peu de temps, une heure ou moins, avant d'être utilisée.

12. Nécessaire contenant essentiellement du facteur GDNF, du fibrinogène, de l'aprotinine et une source d'ions de calcium divalents, pour utilisation dans la préparation d'une composition de colle de fibrine comprenant du facteur GDNF, du fibrinogène, de l'aprotinine et des ions de calcium divalents, destinée à réparer des dommages causés à un nerf du système nerveux central par une maladie ischémique cérébrale ou à favoriser la récupération fonctionnelle du nerf endommagé.

13. Nécessaire conforme à la revendication 12, pour utilisation dans la préparation d'une quantité efficace de composition de colle de fibrine, immédiatement avant son application topique sur un nerf endommagé.

14. Nécessaire conforme à la revendication 12 ou 13, comprenant séparément du facteur GDNF, du fibrinogène, de l'aprotinine et une source d'ions de calcium divalents.
